Europäisches Patentamt

European Patent Office

Office européen des brevets

(19)

(11) Publication number: **0 395 222**
**A2**

(12) # EUROPEAN PATENT APPLICATION

(21) Application number: **90303295.1**

(22) Date of filing: **28.03.90**

(51) Int. Cl.5: **C12M 1/40**

(30) Priority: **27.04.89 GB 8909702**

(43) Date of publication of application:
**31.10.90 Bulletin 90/44**

(84) Designated Contracting States:
**AT BE CH DE FR GB IT LI LU NL SE**

(71) Applicant: **AMERSHAM INTERNATIONAL plc**
**Amersham Place**
**Little Chalfont Buckinghamshire HP7**
**9NA(GB)**

(72) Inventor: **Finlan, Martin Francis**
**7, Ballard Close**
**Aylesbury, Buckinghamshire(GB)**
Inventor: **Garland, Peter Bryan**
**Hope Cottage, Sunnyway**
**Old Bosham, West Sussex PO18 7HQ(GB)**

(74) Representative: **Boydell, John Christopher et al**
**Stevens, Hewlett & Perkins 5 Quality Court**
**Chancery Lane**
**London, WC2A 1HZ(GB)**

(54) **Improvements relating to biosensors.**

(57) A biosensor which can be used for continuous monitoring of biological, biochemical or chemical reactions in which an alternating electrical potential is applied to effect the alternate capture and release of polarisable entities within the sample. There are two basic methods of achieving this, one shown in the attached drawing which shows an SPR (surface plasmon resonance) biosensor in which a sample flowing across a spot (9) of sensitive material in the direction of arrow (A) is subject to an alternating electric field in the area of the reaction (if any) between the sample and the sensitive material. The electric field is generated by applying an alternating potential from a source (12) across the area of the reaction, using an electrode (11).

FIG. 1

## IMPROVEMENTS RELATING TO BIOSENSORS

This invention relates to biosensors for use in biologial, biochemical and chemical testing.

Biosensors are instruments which convert the resultant of an interaction between a sensitive material and a sample to be tested into an electrical signal which can be displayed and/or analysed. In some biosensors the sensitive material comprises molecules of a material which can bind with corresponding molecules in the sample. The presence or absence of binding can be detected and the existence or otherwise of such corresponding molecules in the sample thereby determined. An example is the binding of antibodies with their corresponding antigens. A sensitive material comprising appropriate antibodies can detect the presence or otherwise of antigens in a sample liquid, for example serum. An alternative sensitive material might comprise a DNA or RNA probe which would, during a test, bind (hybridise) with its complement in the sample solution.

It will be appreciated that, once molecules from the sample have become bound to the molecules of the sensitive material, the sensitive material is of no further use, and has to be discarded. This, in the first place, is wasteful of the sensitive material and consumes time because fresh sensitive material has to be presented for each test. Furthermore, continuous testing - for example to detect changes in the composition of a flowing sample material with time - is not practicable.

The biosensor of the present invention provides means for reverse binding of the molecules of the sensitive material. By "reverse binding", we mean that molecules of the sensitive material which have become bound to one or more corresponding molecules from the sample will release those molecules and thus become available for further binding.

In order to provide an instrument which is able sto monitor a flowing sample, it is preferred to provide means for activating the reverse binding means for a period and then de-activating the reverse binding means for a period in a continuous sequence - i .e. on, off, on, off .. . etc.. Thus in a flowing sample, molecules which are released during an active period will be carried away from the sensitive material by the flow of liquid so that, by the end of the active period a "fresh" sensitive material is presented for the next part of the flowing sample to bind (or otherwise) with the molecules of the sensitive material. Means are also provided for monitoring the state of the reaction between the sample and the sensitive material during the de-activated periods when binding is permitted. A suitable monitoring means would be a surface plasmon resonance (SPR) detector such as described in our European Patent Application 0305109.

The rate of the cyclic on/off switching of the reverse binding means depends upon the circumstances. For example, if it were sufficient to take a measurement of a flowing sample once every hour, then the reverse binding means would be activated for most of the time, but with just a short period of reactivation, during which the monitoring means is actuated to detect binding. Alternatively, a more frequent cycling could be envisaged: for example, up to a few hundred Hz.

Two techniques for realising the reverse binding means will now be described:

In the first technique two electrodes are suspended in the sample and an alternating, for example square wave, voltage applied between them. Current flowing through the sample between the electrodes alters the pH of the sample. Provided that the electrodes are suitably arranged with respect to the flow of the sample, the sample can be arranged to take up a pH different from that of the remainder of the solution as it passes the sensitive material. For example, if the liquid is flowed across an electrode which is at a positive potential relative to the other electrode, then the liquid flowing off the electrode will take up a lower pH - i.e. become more acidic. Likewise, if the liquid is flowed across an electrode which is at a negative potential relative to the other electrode, then the liquid flowing off the electrode will take up a higher pH. If, in each case, the sensitive material is positioned to receive the liquid flowing off the first-mentioned electrode, then the pH of the liquid in the area of the sensitive material will be different from that of the remainder. The other electrode is preferably positioned on the downstream side of the sensitive matieral so that it does not influence the pH of the liquid flowing over the sensitive material.

The change in the pH of that portion of the sample immediately adjacent the sensitive material changes the effective charge of that part of the sample with respect to the remainder. This in turn causes the molecules within the solution potentially available for binding to be directed towards, or away from, the sensitive material depending upon the pH of that part of the solution adjacent the sensitive area with respect to the remainder.

Thus, if the potentials applied to the electrodes are reversed in polarity on a cyclic basis (by the application of the aforesaid alternating potential), the molecules in the sample solution will be alternately attracted to, and repelled from, the sensitive surface. During the periods of attraction, molecules in the solution will bind with their complements within the sensitive material; during the periods of repulsion the molecules in the solution will reverse bind from the complements within the sensitive material, and will be repelled away from the sensitive material, both by the electrostatic forces, and by the flow of the sample liquid. Other mechanisms may also come into play which are either in addition to or instead of the mechanism described above. For example, the shape of the epitopes (binding sites) of the molecules either in the sample solution or in the sensitive material, or both, may alter as a result of the changed pH. Thus, molecules which before would have become bound might not now do so, and vice versa.

In the second technique to realise the reverse binding means, it is assumed that the sensitive material is at least partially electrically conductive or is mounted as a thin, for example single molecule thick, layer on a surface which is electrically conductive. A single electrode is provided, and an alternating, for example square wave, voltage is applied between this electrode and the sensitive material, or its conductive mount. If the sample liquid is non conductive, or reasonably so, then an alternating electric field is set up between the electrode and the sensitive material and the sample liquid is caused to flow through this electric field. The electric field acts on any polarised or polarisable entities within the sample to attract them or repel them from the sensitive material, according to the instantaneous direction of the electric field. For example, protein and similar molecules are both inherently polar, and potentially polarisable in the presence of an electric field. Thus, protein molecules can be attracted to and repelled from the sensitive surface, to bind or not as the case may be, in much the same way as described above. As before, other mechanisms may play a part and, indeed several mechanisms may act simultaneously to achieve the desired result: namely the alternate binding and reverse binding of the sample molecules with those of the sensitive material.

It is to be noted that, whilst the invention is described herein in relation to the binding and reverse binding of molecules, it is the case that other entities such as cells and proteins within a given sample solution may be subject to similar forces to those described above, and the invention covers the manipulation of all such polarisable entities. Thus, in its broadest sense, the invention covers the capture and release on an alternating cyclic basis - i.e. capture, release, capture, release .... etc. - of polarisable entities within a sample by a sensitive material, and wherein a parameter of the sensitive material is changed by such capture, means being provided for monitoring that parameter to thereby provide information as to the presence of said entity within the sample.

It is also to be noted that, in referring to a flowing sample, it is not actually necessary for the purpose of this invention for the sample to flow: the use of an alternating binding - reverse binding -binding .... etc. cycle could be applied to separate tests on static samples. Also "flow" includes flow by mechanical means such as pumps, or by convection currents or diffusion, or by any other mechanism which causes the component parts of the sample to move over the sensitive material with the passage of time. The instrument can also be used to monitor static, or flowing, samples whose composition changes with time -for example to directly monitor the progress of a chemical, biochemical or similar reaction.

The exact shape of the alternative waveform used is not thought to be important; the obvious choice is a square wave, with or without a D.C. bias, but a pure sine wave would also work, but possibly less satisfactorily because the speed of binding/reverse binding would be reduced. The mark/space ratio (by which is meant the ratio of the duration of the positive and negative excursions of the waveform) may be altered, depending upon the circumstances. It is likely to be 1:1, particularly in the higher frequency range. The frequency can vary from very low - for example one cycle every few hours, or even longer - to several hundreds of Hz. The likely maximum frequency for the pH change mechanism is several tens of Hz in view of the time taken for the pH to change as it flows across the electrode.

The alternating potential can also act to increase the concentration of the affected species (DNA, polarised protein or whatever) at the active surface of the sensitive material to thus improve the chances that capture of the entities by the sensitive material will occur.

In order that the invention may be better understood, several embodiments thereof will now be described by way of example only, and with reference to the accompanying drawings in which:-

Figures 1 and 2 are diagrammatic views of the two versions of the biosensor according to the invention;

Figure 3 is a waveform diagram, showing examples of the waveform which may be used;

Figure 4 is a side sectional view of a biosensor according to the invention;

Figure 5 is a view on the lines V-V of Figure 4;

Figures 6 and 7 correspond to Figures 4 and 5 respectively, and are of a further embodiment; and

Figure 8 is a view similar to Figure 1, showing an example of a shaped electrode.

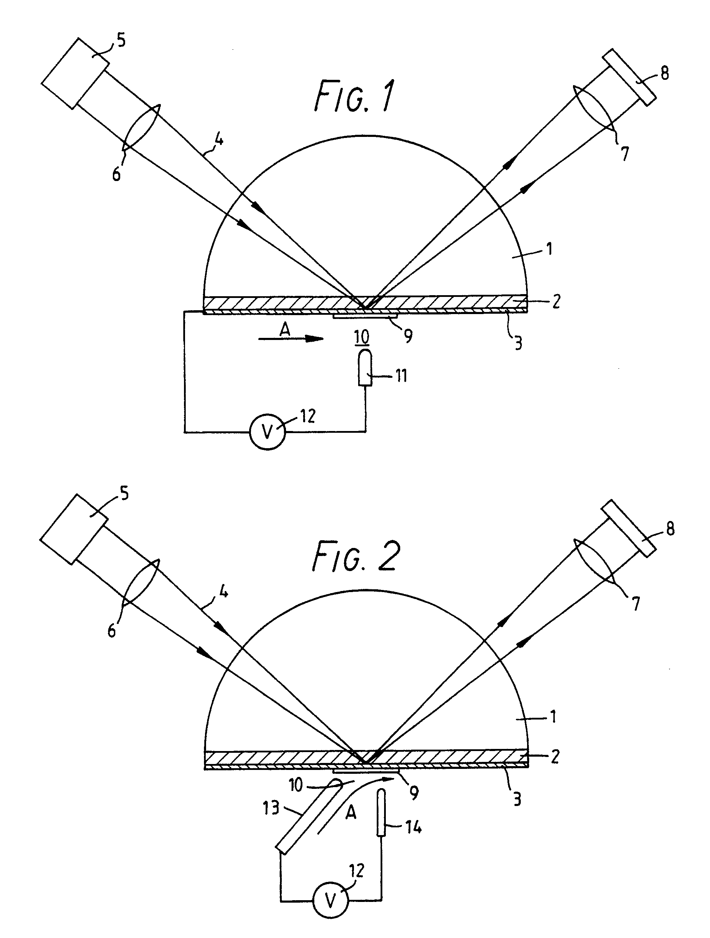

Referring to Figure 1, there is shown an exemplary surface plasmon resonance detector forming part of the biosensor of the present invention.

The biosensor comprises a hemicylindrical or hemispherical prism 1 of glass on the flat surface of which is placed a glass slide 2 of the same refractive index as that of the prism 1. An optical coupling fluid is preferably used between the prism and the slide to improve the optical coupling therebetween. Covering that face of slide 2 remote from the prism 1 is a thin layer 3 of metal such as silver or gold.

P-polarised light 4 from a source 5, for example a laser, is focussed by means of a lens 6 to a point on the plane of the interface between the slide 2 and metal layer 3. In some circumstances, a line focus, extending orthogonally into and out of the plane of Figure 1 may be used.

The light 4 is subject to total internal reflection at the interface between the slide 2 and metal layer 3 and the reflected beam diverges to a second lens 7 from which a parallel light beam emerges, to be incident on a light detector 8.

The arrangement is described in more detail in our European Patent Application 0305109, and will not be repeated here.

Provided that conditions are correct and, in particular that the range of angles of incidence of the incoming beam 4 is appropriate, surface plasmon resonance will occur at the point of reflection, the characteristics of which resonance, as monitored by the strength of the internally reflected beam, are sensitively dependent upon the refractive index of the medium on the opposite face of the metal layer 3 to the slide 2. This "medium" is represented in Figure 1 by a spot 9 of sensitive material. For use in a biosensor, this spot may comprise any suitable material whose reaction with an adjacent sample 10 can be monitored by the change in the refractive index of the spot 9 as the reaction proceeds. For example, the spot 9 may comprise a monolayer of antibodies specific to an expected antigen in the sample. If the antigen is present on the sample, binding occurs, and the resultant refractive index change of the spot 9 can be measured at the detector 8. The spot 9 may comprise a coating of specific material, such as described above, or may be entirely non-specific. A specific coating is one which will bind just one, or possibly a small number, of analytes of interest. A non-specific coating is one which will bind a large number of analytes - for example any protein or any DNA molecule, rather than just a specific one. Indeed the metal layer 3 itself may act as a suitable non-specific material, rendering a separate spot 9 of sensitive material unnecessary.

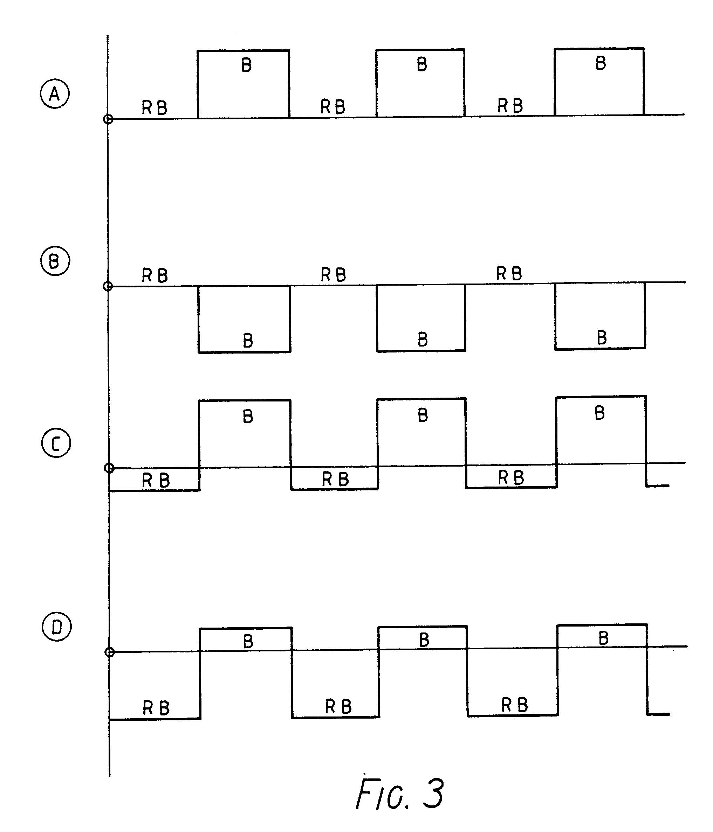

There will now be described the modifications necessary for the present invention. In the first place, although not essential (see above), it is assumed that the sample liquid 10 is flowing. The arrow A indicates this flow diagrammatically. Positioned within the sample liquid and preferably over the spot 9 (or, if there is no spot 9, the point on the metal surface corresponding to the point of total internal reflection) is an electrode 11. A source 12 of alternating potential has output terminals connected respectively to this electrode, and to the metal layer 3 to thereby apply a corresponding alternating electric field between the electrode 11 and the metal layer 3. It is assumed for this purpose that the sample liquid is non-conductive, or substantially so. Examples of the waveforms which may be applied are shown in Figure 3. Typical frequencies are from 1 to a few hundred Hz.

The effect of creating an electric field, of a particular polarity in the area near the spot of sensitive material is to accelerate or switch on the binding of entities - in particular, molecules - in the sample with their corresponding entities in the sensitive material. The effect of an electric field of the opposite polarity is to cause reverse binding of the entities bound to the sensitive material by a previous binding process. The exact mechanism by which these processes occur is discussed above.

It will be seen that an alternating electric field, whose polarity reverses cyclically, will cause alternate binding - reverse binding of entities within the sample with the sensitive material. This is represented by the letters "B" (for binding) and "RB" (for reverse binding) on the graphs in Figure 3; however, these designations should be regarded as exemplary, and could be reversed. During the binding portion of the cycle, the SPR detection system is energised to monitor the binding; during the reverse binding portion of the cycle the previously bound entities are released, and leave the area as a result of the flow of liquid, thus leaving the sensitive material refreshed for the next following binding portion. Thus, a flowing sample can be monitored continuously for changes in the binding behaviour of its molecules.

Figure 2 shows an identical SPR detector with modified sample flow arrangements. In this case, the liquid sample is caused to flow across a first electrode 13 and thence across the spot 9. A second electrode 14 is also positioned within the sample liquid, preferably downstream of the spot 9. The source 12 is connected between electrodes 13 and 14, as shown.

The mechanism by which this version operates is somewhat different to that described with reference to Figure 1, although it should be emphasised that, in any practical realisation of this device, more than one mechanism may operate, depending upon the circumstances. It is assumed that the liquid sample is electrically conductive, or at least partly so, so that, upon application of one of the waveforms of Figure 3 to the electrodes 13/14, a corresponding alternating electric current will flow in the sample from one electrode to the other.

It is known that, when two electrodes are immersed in a conductive liquid and a potential applied, the pH of the liquid in the immediate vicinity of the anode (positive electrode) will fall to a level lower than that of the surrounding liquid, while the liquid in the immediate vicinity of the cathode (negative electrode) will rise. Thus if, as in the arrangement shown, the sample fluid is caused to flow over one of the electrodes (i.e. electrode 13) and then immediately across the spot 9, the sample in the immediate vicinity of the spot 9 will likewise have an altered pH over that of the surrounding liquid. The other electrode (i.e. electrode 14) should be situated in a position in which it cannot influence the pH of the liquid passing over the spot 19 (for example downstream of the spot 9).

Changing the pH of the liquid adjacent the spot 9 causes the overall charge of the liquid in this region to be altered about the isoelectric point with respect to the remainder of the liquid. For example, if the pH of a sample liquid containing analyte (e.g. protein) is appropriate, the overall charge is effectively positive due to the presence of $NH_2$ groups within the molecules. In general terms, the pH of a liquid controls the inherent charge on its molecules.

In the situation where the charge on the molecules in the region of spot 9 is different from that of the molecules in the remainder of the liquid, electric forces will operate to tend to equilibrate the charge and will thus physically move the molecules of the sample liquid towards or away from the area immediately surrounding the spot 9, depending upon the relative polarity of the charges.

Thus an alternating potential such as applied by source 12 will cause alternate movement towards and away of molecules from the spot 9 to thus either assist in binding by forcing molecules into the area of spot 9, or cause or assist in reverse binding by forcing already-bound molecules off the spot 9, back into the liquid.

An apparatus effectively combining the concepts of Figures 1 and 2 may also be envisaged: referring to Figure 2, a potential from a source (not shown) could also be applied between electrodes 13/14 and the metal layer 3 to apply a D.C. potential or an alternating potential synchronised with that from source 12 so as to set up an electric field in the region of spot 9.

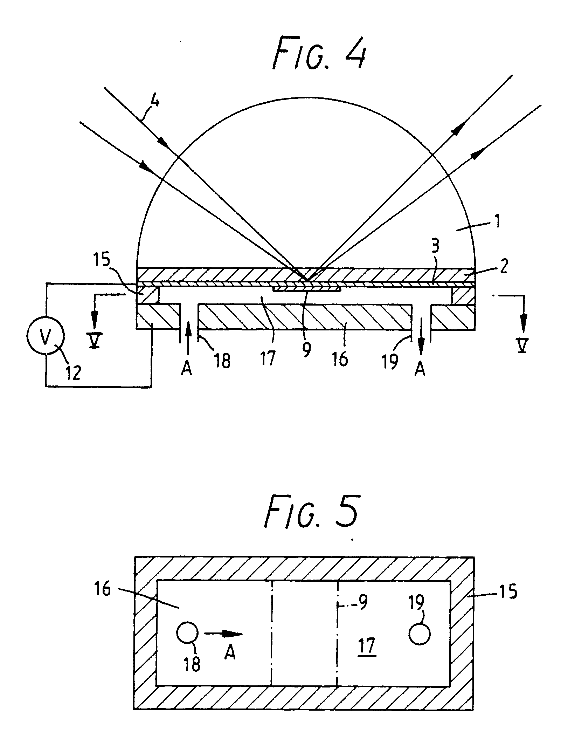

Figures 4 to 7 show two more practicable realisations of the versions illustrated in Figures 1 and 2. Where appropriate, the same reference numerals are used.

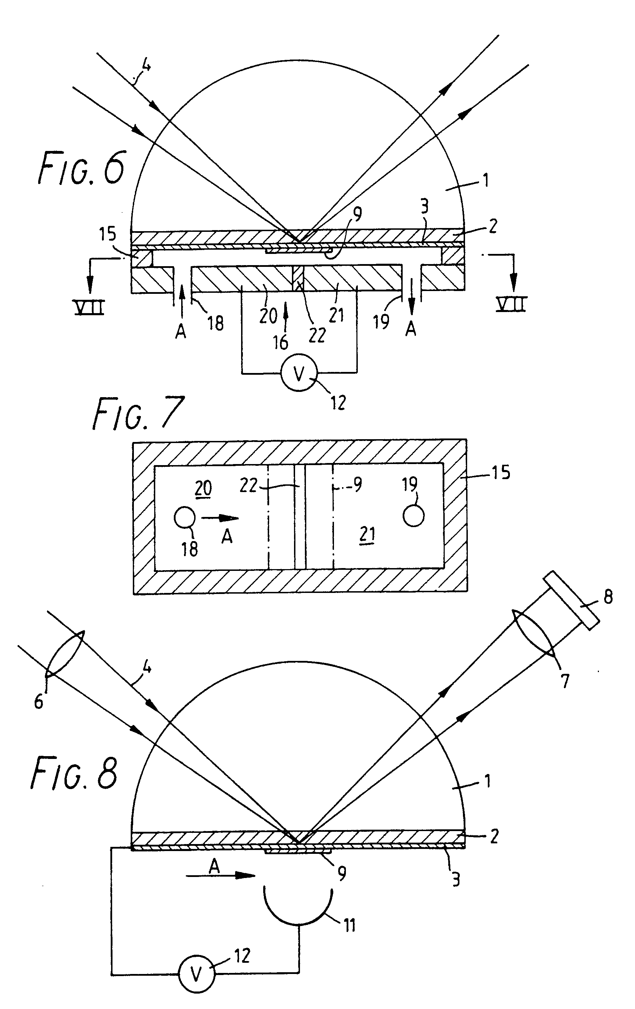

Figures 4 and 5 illustrate a version which operates primarily by means of electric field - i.e. as in Figure 1. The SPR detection arrangements are only partly shown, but are assumed. The lens 1 is hemicylindrical, with an axis extending orthogonally into and out of the plane of Figure 4. The spot 9 of sensitive material is elongate, extending parallel to the lens axis, as illustrated by the dotted lines in Figure 5. Likewise the incoming converging light beam 4 is of wedge shape, the point of incidence at the interface between slide 2 and metal layer 3 actually being a line of incidence also extending parallel to the lens axis. That part of the surface of the metal layer 3 which is not covered by the spot 9 is treated with a substance designed to inhibit non-specific binding of the analyte of interest - for example bovine serum albumin (BSA).

Mounted on the metal layer 3 via a rectangular spacer frame 15 of electrically insulating material is a block 16 of conductive material such as brass. As will be clear from Figure 5, the arrangement defines a rectangular passageway through which sample liquid can flow. An inlet orifice 18 and outlet orifice 19 for sample are provided on the block 16. The direction of flow is illustrated by the arrows A. The rate of flow will be seen to be slowed as it passes through the passageway 17. In a typical arrangement, the length of the passageway 17 is 2cm, its width is 5mm and the diameter of inlet and outlet orifices 18,19 is 1mm. A typical flow rate through passageway 17 is 0.5cm/second.

In order to set up the electric field, the source 12 is connected between the metal layer 3 and the block 16. The block 16 thus acts as an electrode and sets up an electric field, alternating in sympathy with the applied potential, across the passageway 17. This electric field acts in the manner described above to cause alternate binding and reverse binding on a cyclic basis to allow continuous (or intermittent) analysis of a sample flowing through the instrument.

Figures 6 and 7 are views corresponding to 4 and 5 respectively, and show a version which operates primarily by means of pH change - i.e. as in Figure 2. The construction is very similar to that of Figure 1, and only the differences will be noted. The main change is that the metal block 16 of the previous embodiment is divided into two parts 20,21 separated by a layer 22 of electrically insulating material. The source 12 is connected across the two parts 20,21 and thus applies an alternating potential, for example such as shown in Figure 3, across the insulating material. The sample liquid flowing through the cells is assumed to be at least partly conductive so that current is able to flow between parts 20 and 21 via the sample. Thus it will be seen that the two parts 20,21 act in the manner of electrodes 13,14 respectively in the arrangement of Figure 2. As the sample flows across part 20, its molecules take up a charge corresponding to the instantaneous polarity of the part 20, and thus results in that portion of the sample flowing over the sensitive area 9 having an altered pH. This altered pH effects binding or reverse binding (according to the·instantaneous polarity) by the mechanism discussed above.

Referring again to Figure 1, the shape of electrode 11 can be modified to provide a more concentrated electric field and/or a particular profile of electric field in the region of the spot 9 of sensitive material. An example of a hemicylindrical or hemispherical electrode 11 is illustrated in Figure 8. This acts to concentrate the lines of force in the immediate area of the SPR influence, and thus enhances the binding/reverse binding effect in this area, thus improving the sensitivity of the instrument. Other methods of concentrating the field and/or modifying the field pattern to suit the circumstances will be apparent to those skilled in the art.

In all of the above-described embodiments, the spot 9 comprises a layer of sensitive material. This sensitive material may be any material which is capable of interacting in some way with a component of a sample liquid so as to bring about a change in a property or properties of spot 9 which can be detected - in all of the embodiments described herein the relevant property is the refractive index of the layer comprising the spot 9 which is monitored by means of surface plasmon resonance. Needless to say, the entity within the sample liquid which interacts with the material of the spot 9 must be one which is effected by one or more of the mechanisms described herein - pH change, electrostatic forces or whatever.

The sensitive material comprising the spot 9 may be specific to a particular entity within the sample or may be non-specific (i.e. may interact with several species of entity within the sample). Examples of specific materials include recognition molecules such as antibodies which will specifically bind an analyte of interest within the sample, DNA/RNA probes which will bind with their complements in the sample liquid, or lectins, glycoproteins or enzyme substrates, all of which are capable of recognising and binding with the other partner in a bimolecular recognition pair.

Examples of non-specific materials include hydrophobic materials, for example in the form of a monolayer of phospholipid-type molecules to capture amphipathic molecules, or hydrophilic materials which would capture polysaccharides. Indeed, it has been found that the surface of the metal layer itself can form an effective non-specific binding material. Silver or gold surfaces will bind proteins or polynucleotides such as DNA or RNA without the need for any further coating and, in this case the spot 9 is effectively dispensed with altogether, and the surface of layer 3 used directly for binding. This apparatus can thus be used to monitor, for example, protein concentration in a sample and, in this role is both fast (few minutes), sensitive (submicrogram), needs only a few microlitres of sample to test, and requires no reagents.

The above mechanisms can also be aided by electrolytic or amperometric effects to generate chemical species at or near the surface of metal layer 3. Such effects may also be used independently for example in catalysing or generating enhancement effects.

For example, it is known that living cell processes may be monitored by analysing the metabolites, which latter are often oxidisable or reduceable. Thus, taking the generalised Figure 2 arrangement, the sample can be arranged to flow over living cells, prior to entering the Figure 2 device, in order to collect metabolites. The sample, now containing metabolites, enters the Figure 2 device and the resultant change of pH will oxidise or reduce (depending upon the polarity of electrode 13) the metabolites and therefore cause binding or reverse binding with a binding agent, for example antibody, forming spot 9.

As a general matter, it is possible for the electrodes to produce an oxidising or reducing environment which can result in deposition of polymer on the spot 9. This ability to provide an oxidising/reducing environment provides the facility of being able to switch on or off a number of SPR enhancement processes.

## Claims

1. A biosensor for monitoring the interaction between a sensitive material and a sample to be tested, said biosensor comprising means for effecting the alternate capture and release on an alternating cyclic basis of polarised or polarisable entities within the sample by the sensitive material such that a parameter of the sensitive material is changed by such capture, and means for monitoring said parameter to provide information as to the presence and/or concentration of said entity within the sample.

2. A biosensor as claimed in claim 1 wherein the means for effecting alternate capture and release comprises means for reverse binding of the molecules of the sensitive material (as herein defined), together with means for activating and deactivating said reverse binding means on a cyclic basis.

3. A biosensor as claimed in either one of claims 1 or 2 wherein the means for effecting alternate capture and release includes a source of alternating voltage applied in such a way as to cause capture of the polarisable entities over a first part of each cycle of the alternating voltage and to cause release of the polarisable entities over a second part of each cycle of the alternating voltage.

4. A biosensor as claimed in claim 3 wherein said alternating voltage alternates about a base potential, and wherein said first part of each cycle is that part of the cycle on one side of said base potential and said second part of each cycle is that part of the cycle on the other side of said base potential.

5. A biosensor as claimed in either one of claims 3 or 4 wherein the length (in time) of said one part of each cycle is different to the length of the other part of each cycle.

6. A biosensor as claimed in any one of claims 3 to 5 wherein the sample fluid is electrically conductive or partially conductive, and wherein said alternating voltage is applied in such a way as to cause a corresponding alternating flow of current in said sample in the vicintiy of the interaction between the sample and the sensitive layer.

7. A biosensor as claimed in claim 6 wherein at least two electrodes are positioned in contact with the sample, said source of alternating voltage being connected to said electrodes so as to evoke a corresponding alternating current to flow in the sample between the electrodes.

8. A biosensor as claimed in claim 7 wherein means are provided for causing the sample to flow across the area of interaction between it and the sensitive material, and wherein one of said electrodes is placed on the upstream side of the area of interaction, and one on the downstream side.

9. A biosensor as claimed in claim 8 wherein said one (upstream) electrode is positioned immediately adjacent the· area of interaction and the sample is caused to flow across said one electrode and thence immediately into said area of interaction.

10. A biosensor as claimed in any one of claims 3 to 5 wherein the sample fluid is substantially nonconductive, and wherein said alternating voltage is applied in such a way as to set up an alternating electric field in the sample in the area of interaction between it and the sensitive material.

11. A biosensor as claimed in claim 10 wherein the electric field is established between a pair of electrodes to which is connected said source of alternating voltage.

12. A biosensor as claimed in claim 11 wherein the sensitive material is at least partially electrically conductive and acts as one of said pair of electrodes.

13. A biosensor as claimed in claim 12 wherein the sensitive material is mounted as a thin layer on a conductive surface and wherein said conductive surface acts as one of said pair of electrodes.

14. A biosensor as claimed in either one of claims 12 or 13 wherein the other of said pair of electrodes is suspended in the sample.

15. A biosensor as claimed in any one of the preceding claims including pump means for causing the sample to flow over the sensitive material.

16. A method of monitoring the interaction between a sensitive material and a sample to be tested, said method comprising changing a parameter of said sensitive material by causing said sensitive material to alternately and cyclically capture and release polarised or polarisable entities within the sample, and monitoring said parameter to provide information as to the presence and/or concentration of said entity within the sample.

EP 0 395 222 A2

FIG. 1

FIG. 2

FIG. 3

FIG. 4

FIG. 5

FIG. 6

FIG. 7

FIG. 8